# EUROPÄISCHE PATENTSCHRIFT

(11) **EP 3 011 914 B1**
(45) Veröffentlichungstag und Bekanntmachung des Hinweises auf die Patenterteilung: **19.09.2018**
(21) Anmeldenummer: 15190358.0
(22) Anmeldetag: 19.10.2015
(51) Int. Cl.: A61B 17/08, A61B 17/10

(54) **MEDIZINISCHES PRODUKT UND MEDIZINISCHES KIT ZUR ANWENDUNG BEIM VERSCHLUSS VON BIOLOGISCHEM GEWEBE**
MEDICAL PRODUCT AND MEDICAL KIT FOR USE IN THE CLOSURE OF BIOLOGICAL TISSUE
PRODUIT MEDICAL ET KIT MEDICAL DESTINES A ETRE UTILISES LORS DE LA FERMETURE DE TISSUS BIOLOGIQUES

(30) Priorität: 20.10.2014 DE 102014221259
(43) Veröffentlichungstag der Anmeldung: 27.04.2016
(73) Patentinhaber: AESCULAP AG, 78532 Tuttlingen (DE)
(72) Erfinder: Mattes, Ursula, 78603 Renquishausen (DE)
(74) Vertreter: Patentanwälte Ruff, Wilhelm, Beier, Dauster & Partner mbB

(56) Entgegenhaltungen:
- DE-C- 153 744
- US-A1- 2012 109 159
- US-A1- 2013 116 794
- US-A1- 2014 012 192
- US-A1- 2014 128 819

## Beschreibung

### Anwendungsgebiet und Stand der Technik

Die Erfindung betrifft ein medizinisches Produkt sowie ein medizinisches Kit.

Die minimalinvasive Chirurgie ("Schlüsselloch-Chirurgie") stellt eine moderne Operationstechnik dar, bei welcher die Operation mehr oder weniger innerhalb der geschlossenen Bauchhöhle oder Brusthöhle stattfindet. Große für den Patienten belastende Bauch- oder Brustwandschnitte entfallen weitgehend. Stattdessen sind lediglich kleine, kaum sichtbare Einschnitte zum Einführen eines Endoskops erforderlich.

Obgleich die minimalinvasive Chirurgie seit mittlerweile mehr als 25 Jahren praktiziert wird, stellt die endoskopische Naht für die meisten Chirurgen noch immer eine große Herausforderung dar. Die klassische Naht wird mit Hilfe von zwei Endo-Nadelhaltern sowie einem konventionellen Nahtmaterial erzeugt. Diese Technik ist sehr zeitaufwändig und erfordert ein intensives Training sowie eine kontinuierliche Übung, weswegen sie bei vielen Chirurgen nur wenig Akzeptanz findet. Automatisierte sowie halbautomatisierte Verfahren konnten sich bislang aufgrund von patientenindividuellen Gewebestrukturen nicht durchsetzen.

Ein medizinisches Produkt zur Approximation und Fixierung von Gewebe, wobei das Produkt einen hohlen Schaft sowie ein darin angeordnetes Fixierungsmittel aufweist, ist aus der US 2012/0109159 A1 bekannt.

Ein als Gewebebrücke wirkendes medizinisches Produkt zur Approximation und Fixierung von Gewebe ist aus der US 2014/0128819 A1 bekannt.

Aus der US 2013/0116794 A1 ist eine Prothese für das Schultergelenk bekannt, welche mittels einer flexiblen Umhüllung in einem zusammengerollten Zustand gehalten werden kann.

Aus der DE 153 744 C ist eine Vorrichtung zum Anlegen von Wundklammern mit schleifenartig umgebogenen Enden bekannt.

Ein medizinisches Produkt mit einer flexiblen, offen ringförmigen Struktur ist aus der US 2014/0012192 A1 bekannt.

### Aufgabe und Lösung

Der Erfindung lag die Aufgabe zugrunde, ein medizinisches Produkt bereitzustellen, welches sich allgemein zum Verschluss von Körpergewebe eignet und aus dem Stand der Technik bekannte Nachteile vermeidet. Das medizinische Produkt soll insbesondere einen schnellen, einfachen sowie risikoarmen Verschluss von Körpergewebe ermöglichen.

Diese Aufgabe wird gelöst durch ein medizinisches Produkt mit den Merkmalen des unabhängigen Anspruchs 1 sowie durch ein Kit mit den Merkmalen von Anspruch 13. Bevorzugte Ausführungsformen sind in den abhängigen Ansprüchen 2 bis 12 definiert. Der Wortlaut sämtlicher Ansprüche wird hiermit durch ausdrückliche Bezugnahme zum Inhalt der vorliegenden Beschreibung gemacht.

In einem ersten Aspekt wird die der Erfindung zugrundeliegende Aufgabe durch ein medizinisches Produkt gelöst, welches ein wenigstens teilweise zusammengerolltes Flächengebilde aufweist. Das Flächengebilde ist unter wenigstens teilweiser Aufhebung einer Zusammenrollkraft in einen aufgeweiteten bzw. aufgespreizten, insbesondere auseinandergerollten, Zustand überführbar, vorzugsweise vorspannbar. Bei dem Flächengebilde handelt es sich um ein flexibles Flächengebilde.

Das medizinische Produkt zeichnet sich besonders dadurch aus, dass das Flächengebilde Verankerungselemente zur Verankerung des Flächengebildes und damit des medizinischen Produktes in einem biologischen, insbesondere menschlichen oder tierischen, Gewebe aufweist.

Das medizinische Produkt ist vorzugsweise zur Anwendung beim Verschluss, d.h. zur Anwendung bei der Approximation und Fixierung, von biologischem, insbesondere menschlichem oder tierischem, Gewebe vorgesehen.

Bevorzugt handelt es sich bei dem Gewebe im Sinne der vorliegenden Erfindung um menschliches oder tierisches Körpergewebe, wobei Körpergewebe menschlicher Patienten besonders bevorzugt ist.

Das medizinische Produkt ist weiterhin bevorzugt zur Anwendung beim Verschluss von Wunden, insbesondere inneren Wunden, vorgesehen.

Unter dem Ausdruck "Zusammenrollkraft" soll im Sinne der vorliegenden Erfindung eine Art Rückstellkraft verstanden werden, welche auf das Flächengebilde in einem aufgeweiteten bzw. aufgespreizten, insbesondere auseinandergerollten, Zustand wirkt und das Flächengebilde wieder in einen wenigstens teilweise zusammengerollten Zustand "zurückstellen" möchte.

Das erfindungsgemäße Produkt, welches unter funktionalen Gesichtspunkten als eine Art Roll-Clip bezeichnet werden kann, wird im aufgeweiteten bzw. aufgespreizten, insbesondere auseinandergerollten, Zustand des Flächengebildes auf ein zu verschließendes Gewebe appliziert. Ein solches Gewebe zeichnet sich durch (wenigstens) einen Gewebespalt aus, entlang dessen Gewebebereiche voneinander getrennt vorliegen. Die Verankerungselemente dringen nach erfolgter Applikation des Produktes in das Gewebe ein und bewirken eine Verankerung des Flächengebildes und folglich des Produktes im Gewebe. Die Applizierung des Produktes geschieht in der Regel mittels eines Applikators, welcher dazu ausgebildet ist, das Flächengebilde im aufgeweiteten bzw. aufgespreizten, insbesondere auseinandergerollten, Zustand zu halten. Auf einen entsprechenden Applikator wird im Folgenden noch näher eingegangen werden. Nach Wegnahme des Applikators rollt sich das Flächengebilde, der "rückstellenden" Zusammenrollkraft folgend, zusammen. Über die Verankerungselemente, welche aufgrund der Zusammenrollbewegung des Flächengebildes mitbewegt werden, werden die durchtrennten Gewebebereiche ausgehend von ihren gewebespaltseitigen Enden in dem Flächengebilde aufgerollt bzw. aufgewickelt und gleichzeitig aneinander fixiert. Dadurch ist ein dauerhafter Gewebeverschluss erzielbar. Im Ergebnis bewirkt somit die auf das Flächengebilde im aufgeweiteten bzw. aufgespreizten, insbesondere auseinandergerollten, Zustand wirkende Zusammenrollkraft den Gewebeverschluss.

Das erfindungsgemäße Produkt bietet dem Chirurgen eine einfache, schnelle und risikoarme Möglichkeit zur Herbeiführung eines Gewebeverschlusses. Ein komplizierter chirurgischer Eingriff, insbesondere unter Verwendung von Endo-Nadelhaltern und konventionellen Nahtmaterialien, ist nicht erforderlich. Nahtmaterialbedingte Behandlungsfehler können mithin vermieden werden. Weitere Vorteile bestehen in der Verkürzung von Operationszeiten sowie dem Wegfall eines aufwendigen Trainings zum Erlernen von komplizierten Nähten, wie beispielsweise endoskopischen Nähten. Zur weiteren Risikominimierung kann es von Vorteil sein, wenn das medizinische Produkt mittels eines hierfür geeigneten Applikators appliziert wird. Ein solcher Applikator bietet zudem die Vorteile einer besonders exakten und insbesondere schnellen Positionierung des medizinischen Produktes auf einem zu verschließenden Gewebe.

Das Flächengebilde weist zwei zusammengerollte Teilbereiche auf. Die beiden Teilbereiche sind vorzugsweise zylindrisch, insbesondere kreiszylindrisch, ausgebildet.

Bevorzugt sind die zwei Teilbereiche ausgehend von einem noch nicht zusammengerollten Flächengebilde mittels Zusammenrollen ausgehend von zwei gegenüberliegenden Seitenrändern, vorzugsweise Querseitenrändern, des Flächengebildes gebildet.

In einer weiteren Ausführungsform berühren sich die zwei Teilbereiche gegenseitig. Erfindungsgemäß ist es insbesondere bevorzugt, wenn die zwei Teilbereiche aufgrund der Zusammenrollkraft aneinander gedrückt, insbesondere aneinander gepresst, werden.

Erfindungsgemäß ist es weiterhin bevorzugt, wenn das Flächengebilde zwischen den zwei zusammengerollten Teilbereichen einen nicht zusammengerollten Zwischenbereich aufweist. Der Zwischenbereich kann eben, d.h. nicht gewölbt, oder gewölbt ausgebildet sein.

Das Flächengebilde kann im aufgeweiteten bzw. aufgespreizten, insbesondere auseinandergerollten, Zustand eine polygonale (vieleckige) Form besitzen. Beispielsweise kann das Flächengebilde in diesem Zustand eine polygonale Form besitzen, die ausgewählt ist aus der Gruppe bestehend aus trigonale Form, tetragonale, insbesondere quadratische oder rechteckförmige, Form, pentagonale Form, hexagonale Form, heptagonale Form, oktogonale Form, enneagonale Form, dekagonale Form, hendekagonale Form und dodekagonale Form.

In einer alternativen Ausführungsform besitzt das Flächengebilde im aufgeweiteten bzw. aufgespreizten, insbesondere auseinandergerollten, Zustand eine ovale, d.h. eine rundliche konvexe, Form. Beispielsweise kann das Flächengebilde eine kreisförmige oder elliptische Form besitzen.

Bei dem Flächengebilde handelt es sich um ein textiles Flächengebilde, d.h. um ein Flächengebilde, welches aus Fäden gebildet ist. Bei den Fäden kann es sich um Polymer- und/oder Metallfäden handeln. Bezüglich geeigneter Polymere und Metalle wird auf die im Folgenden im Zusammenhang mit dem Flächengebilde sowie den Verankerungselementen genannten Materialien Bezug genommen.

Das Flächengebilde kann grundsätzlich als Gewebe, Gewirk, Gestrick, Geflecht, Vlies oder Gelege ausgestaltet sein.

In einer besonders bevorzugten Ausführungsform handelt es sich bei dem Flächengebilde um ein Netz, insbesondere gewirktes Netz.

Alternativ handelt es sich bei dem Flächengebilde um ein Gitter.

Das in den beiden vorherigen Absätzen erwähnte Netz bzw. Gitter kann eine wabenförmige Struktur, d. h. eine Struktur mit hexagonalförmigen Öffnungen, besitzen.

Grundsätzlich können das Flächengebilde und die Verankerungselemente das gleiche Material aufweisen oder aus dem gleichen Material gebildet sein. Alternativ können das Flächengebilde und die Verankerungselemente verschiedene Materialien aufweisen oder aus verschiedenen Materialien gebildet sein. Bezüglich geeigneter Materialien wird auf die folgenden Ausführungsformen verwiesen.

Das Flächengebilde und/oder die Verankerungselemente können grundsätzlich ein nicht resorbierbares, resorbierbares oder teilresorbierbares Material aufweisen oder aus einem solchen Material gebildet sein. Das Material kann ausgewählt sein aus der Gruppe bestehend aus Polymere, synthetische Polymere bzw. Kunststoffe, Biopolymere, Metalle bzw. metallische Werkstoffe, Metalllegierungen, Keramiken und Kombinationen, insbesondere Komposite bzw. Verbundmaterialien, davon.

Das Flächengebilde und/oder die Verankerungselemente können beispielsweise ein nicht resorbierbares Polymer aufweisen oder aus einem nicht resorbierbaren Polymer gebildet sein, welches ausgewählt ist aus der Gruppe bestehend aus Polyethylen, Polyethylen mit niedriger Dichte, Polyethylen mit hoher Dichte, hochmolekulares Polyethylen, ultrahochmolekulares Polyethylen, Polypropylen, Polyethylenterephthalat, Polypropylenterephthalat, Polybutylenterephthalat, Polyacrylnitril, Polyamid 6, Polyamid 6-6, Polyamid 6-12, Polyamid 12, Polytetrafluorethylen, insbesondere expandiertes Polytetrafluorethylen, Polyvinylidendifluorid, Polytetrafluorpropylen, Polyhexafluorpropylen, Elastomere, thermoplastische Elastomere, Formgedächtnispolymere, Copolymere davon und Kombinationen, insbesondere Komposite bzw. Verbundmaterialien, davon.

In einer weiteren Ausführungsform weisen das Flächengebilde und/oder die Verankerungselemente ein resorbierbares Polymer auf oder sind aus einem resorbierbaren Polymer gebildet, welches ausgewählt ist aus der Gruppe bestehend aus Polyhydroxyalkanoate, Polyglycolsäure, Polymilchsäure, Poly-3-hydroxybuttersäure, Poly-4-hydroxybuttersäure, Poly-ε-caprolacton, Polydioxanon, Polytrimethylencarbonat, Poly-ε-caprolacton, Polyvinylalkohol, Salze davon, Copolymere davon und Kombinationen, insbesondere Komposite bzw. Verbundmaterialien, davon.

Weiterhin können das Flächengebilde und/oder die Verankerungselemente ein Metall/eine Metalllegierung aufweisen oder aus einem Metall/einer Metalllegierung gebildet sein. Bei dem Metall/der Metalllegierung kann es sich insbesondere um ein Formgedächtnismetall/eine Formgedächtnislegierung handeln. Das Metall/die Metalllegierung kann beispielsweise ausgewählt sein aus der Gruppe bestehend aus Eisen, Chrom, Nickel, Molybdän, Titan, Kobalt, Mangan, Tantal, Nitinol und Legierungen davon.

In einer bevorzugten Ausführung weisen das Flächengebilde und/oder die Verankerungselemente einen Stahl auf oder sind aus einem Stahl gebildet. Bei dem Stahl kann es sich insbesondere um einen Edelstahl handeln. Bevorzugt weist der Stahl neben Eisen wenigstens eine weitere metallische Komponente auf, welche ausgewählt ist aus der Gruppe bestehend aus Chrom, Nickel, Molybdän, Titan, Niob, Aluminium, Wolfram und Kobalt. Entsprechend ist es bevorzugt, wenn es sich bei dem Stahl um einen Chromstahl, Chrom-Nickel-Stahl, Chrom-Molybdän-Stahl oder Titanstahl handelt. Beispielsweise kann es sich bei dem Stahl um einen Chrom-Nickel-Stahl mit einem Kohlenstoffgehalt von 0.05 Gew.-% bis 0.15 Gew.-%, einem Chromgehalt von 16 Gew.-% bis 19 Gew.-%, einem Nickelgehalt von 6 Gew.-% bis 9.50 Gew.-%, einem Molybdängehalt von maximal 0.80 Gew.-% sowie einem Stickstoffgehalt von maximal 0.11 Gew.-% handeln. Ein solcher Chrom-Nickel-Stahl ist unter der Werkstoffnummer WNr. 1.4310 (X10CrNi18-8) erhältlich.

Ist das Flächengebilde als textiles Flächengebilde gefertigt, kann es bevorzugt sein, wenn das Flächengebilde Bikomponentenfäden aufweist oder aus solchen Fäden gebildet ist. Beispielsweise können die Fäden einen Kern bzw. eine Seele aus Metall und eine Ummantelung aus einem Polymer (Kern-Mantel-Fäden) besitzen. Bezüglich geeigneter Polymere und Metalle wird auf die zuvor im Zusammenhang mit dem Flächengebilde sowie den Verankerungselementen genannten Materialien Bezug genommen.

Das Flächengebilde und die Verankerungselemente können monolithisch, d.h. einstückig, ausgebildet sein. Eine solche Ausbildung lässt sich beispielsweise mittels Spritzgießen realisieren. Alternativ können das Flächengebilde und die Verankerungselemente miteinander verbunden sein, d.h. der Zusammenhalt zwischen dem Flächengebilde und den Verankerungselementen kann auf einer Fügeverbindung beruhen. Obgleich eine form- und/oder kraftschlüssige Verbindung zwischen dem Flächengebilde und den Verankerungselementen grundsätzlich möglich sein können, ist es bevorzugt, wenn das Flächengebilde und die Verankerungselemente stoffschlüssig miteinander verbunden sind. Beispielsweise können die Verankerungselemente mit dem Flächengebilde verklebt, verschweißt und/oder verlötet sein.

Die Verankerungselemente sind in einer weiteren Ausführungsform in Form von Haken, insbesondere Widerhaken, Ankern, Krallen, Klammern, Zähne und Kombinationen davon ausgebildet.

Erfindungsgemäß kann es beispielsweise vorgesehen sein, dass die Verankerungselemente in Form wenigstens einer Zahnreihe ausgebildet sind. Erfindungsgemäß kann es ggf. weiterhin vorgesehen sein, dass die wenigstens eine Zahnreihe zusätzlich zu anders ausgestalteten Verankerungselementen, wie beispielsweise im vorherigen Absatz genannt, zur Unterstützung des Gewebeverschlusses vorgesehen ist.

Wie bereits erwähnt, weist das Flächengebilde zwei zusammengerollte und vorzugsweise zylinderförmig ausgebildete Teilbereiche auf, wobei das Flächengebilde zwischen den Teilbereichen einen nicht zusammengerollten Zwischenbereich aufweisen kann. Die Verankerungselemente sind dabei vorzugsweise nur in den zusammengerollten Teilbereichen, und zwar nur entlang einer Seite dieser Teilbereiche, ausgebildet, wohingegen der Zwischenbereich vorzugsweise frei von Verankerungselementen ist. Bezüglich weiterer Merkmale der Teilbereiche sowie des Zwischenbereiches wird vollständig auf die bislang gemachten Ausführungen Bezug genommen.

Die Verankerungselemente des Flächengebildes sind nur auf einer Seitenfläche des Flächengebildes ausgebildet, wohingegen eine vorzugsweise gegenüberliegende Seitenfläche des Flächengebildes frei von Verankerungselementen ist. Unter den Seitenflächen werden im Sinne der vorliegenden Erfindung die beiden größten, vorzugsweise gegenüberliegenden Flächen des Flächengebildes verstanden.

Insbesondere können die Verankerungselemente nur randseitig auf einer Seitenfläche des Flächengebildes ausgebildet sein.

Die Verankerungselemente sind auf Teilflächen einer Seitenfläche des Flächengebildes ausgebildet, welche im aufgeweiteten bzw. aufgespreizten, insbesondere auseinandergerollten, Zustand des Flächengebildes gegenüberliegend angeordnet sind. Bei den Teilflächen handelt es sich um randseitig verlaufende Teilflächen, d.h. Randflächen, der Seitenfläche. Besonders bevorzugt erstrecken sich die Teilflächen entlang von vorzugsweise gegenüberliegenden Querseiten der Seitenfläche. Unter Querseiten sollen im Sinne der vorliegenden Erfindung die sich in Querrichtung der Seitenfläche erstreckenden Seiten verstanden werden.

Um das Eindringen der Verankerungselemente in ein zu verschließendes Gewebe zu erleichtern, stehen die Verankerungselemente in einer weiteren Ausführungsform von der Oberfläche des Flächengebildes ab. Mit anderen Worten ist es in einer zweckmäßigen Ausführungsform vorgesehen, dass die Verankerungselemente in Form von Vorsprüngen auf der Oberfläche des Flächengebildes ausgebildet sind. Dabei können die Verankerungselemente rechtwinklig und/oder in spitzen Winkeln von dem Flächengebilde abstehen.

In einer weiteren unter Verankerungsgesichtspunkten vorteilhaften Ausführungsform besitzt das Flächengebilde im aufgeweiteten bzw. aufgespreizten, insbesondere auseinandergerollten, Zustand gekrümmte, gewölbte oder umgebogene Randflächen, auf welchen die Verankerungselemente vorzugsweise ausgebildet sind. Bei den Randflächen handelt es sich bevorzugt um gegenüberliegende Seitenrandflächen, besonders bevorzugt Querseitenrandflächen, des Flächengebildes.

In einer weiteren Ausführungsform ist das Flächengebilde wenigstens teilweise mit einer Beschichtung versehen. Die Beschichtung kann dabei unterschiedliche Funktionen erfüllen.

Beispielsweise kann eine Beschichtung des Flächengebildes vorgesehen sein, um die im aufgeweiteten bzw. aufgespreizten, insbesondere auseinandergerollten, Zustand auf das Flächengebilde wirkende Zusammenrollkraft zu erhöhen. Bezüglich geeigneter Beschichtungsmaterialien wird auf die im Zusammenhang mit dem Flächengebilde und den Verankerungselementen genannten Materialien Bezug genommen.

Weiterhin kann die Beschichtung den Zweck einer Wirkstofffreisetzung erfüllen. Entsprechend kann es von Vorteil sein, wenn die Beschichtung medizinische Wirkstoffe aufweist, welche vorzugsweise ausgewählt sind aus der Gruppe bestehend aus wundheilungsfördernde Wirkstoffe, blutstillende Wirkstoffe, entzündungshemmende Wirkstoffe, antimikrobielle, insbesondere antibiotische, Wirkstoffe, schmerzlindernde Wirkstoffe und Mischungen davon.

Das medizinische Produkt ist zur Anwendung als chirurgisches Implantat vorgesehen. Mit anderen Worten handelt es sich bei dem medizinischen Produkt gemäß der vorliegenden Erfindung um ein chirurgisches Implantat.

Das medizinische Produkt kann insbesondere zum Ersatz einer endoskopischen Naht oder von chirurgischen Klammern vorgesehen sein.

In einer weiteren Ausführungsform liegt das medizinische Produkt in Form eines Flächengebildes vor, wobei das Flächengebilde Verankerungselemente zur Verankerung in einem biologischen, insbesondere menschlichen oder tierischen, Gewebe aufweist. Bezüglich weiterer Merkmale und Vorteile des Flächengebildes sowie der Verankerungselemente wird vollständig auf die bisherige Beschreibung Bezug genommen.

Gemäß einem zweiten Aspekt betrifft die Erfindung ein medizinisches Kit, insbesondere zur Anwendung beim Verschluss, d.h. zur Anwendung bei der Approximation und Fixierung, von biologischem, insbesondere menschlichem oder tierischem, Gewebe.

Besonders bevorzugt ist das Kit zur Anwendung beim Verschluss von Wunden, insbesondere inneren Wunden, vorgesehen.

Das medizinische Kit weist ein medizinisches Produkt gemäß erstem Erfindungsaspekt sowie wenigstens eine weitere Kitkomponente auf. Die wenigstens eine weitere Kitkomponente ist ausgewählt aus der Gruppe bestehend aus einem Applikator für das medizinische Produkt und einem medizinisch verträglichen Kleber.

Der Applikator ist vorzugsweise dazu ausgebildet, das Flächengebilde des medizinischen Produktes in einen aufgeweiteten bzw. aufgespreizten, insbesondere auseinandergerollten, Zustand zu überführen, vorzugsweise vorzuspannen. Zweckmäßigerweise weist der Applikator hierfür eine entsprechend ausgebildete Anlegefläche auf. Bevorzugt ist eine auf- und zusammenklappbare Anlegefläche. Der Applikator kann somit funktional als Aufweit- bzw. Spreizapplikator bezeichnet werden.

In einer weiteren Ausführungsform ist der Applikator derart ausgebildet, dass ein Applizieren mehrerer erfindungsgemäßer Produkte innerhalb kürzester Zeit möglich ist. Der Applikator kann hierzu beispielsweise ein Magazinrohr aufweisen, mittels welchem mehrere erfindungsgemäße Produkte bis zu ihrer Applikation bevorratet werden können. Erfindungsgemäß kann es insbesondere vorgesehen sein, dass das Magazinrohr bereits mehrere erfindungsgemäße Produkte enthält.

Obgleich für einen sicheren Gewebeverschluss an sich nicht erforderlich, kann das erfindungsgemäße Kit, wie bereits erwähnt, einen medizinisch verträglichen Kleber umfassen. Auf diese Weise ist eine zusätzliche Absicherung des herbeizuführenden Gewebeverschlusses über eine Verklebung möglich.

Bei dem medizinisch verträglichen Kleber kann es sich beispielsweise um einen cyanacrylathaltigen Kleber handeln. Ein geeigneter cyanacrylathaltiger Kleber wird von der Anmelderin beispielsweise unter der Bezeichnung Histoacryl® kommerziell vertrieben. Dieser Kleber basiert auf N-Butylcyanacrylat-Monomeren. Alternativ kann es sich bei dem medizinisch verträglichen Kleber um einen Zweikomponenten-Kleber, wie beispielsweise um einen Fibrinkleber, handeln.

Bezüglich weiterer Merkmale und Vorteile des medizinischen Kits, insbesondere des medizinischen Produktes, wird zur Vermeidung von Wiederholungen vollständig auf die im Rahmen des ersten Erfindungsaspektes gemachten Ausführungen Bezug genommen.

Gemäß einem dritten Aspekt wird ein weiteres medizinisches Kit, insbesondere zur Anwendung beim Verschluss, d.h. zur Anwendung bei der Approximation und Fixierung, von biologischem, insbesondere menschlichem oder tierischem, Gewebe offenbart

Das medizinische Kit weist ein medizinisches Produkt und einen Applikator auf. Das medizinische Produkt selbst weist ein vorzugsweise flexibles Flächengebilde mit Verankerungselementen zur Verankerung in einem biologischen, insbesondere menschlichen oder tierischen, Gewebe auf und ist mittels des Applikator unter wenigstens teilweiser Aufhebung einer Zusammenrollkraft in einen aufgeweiteten bzw. aufgespreizten, insbesondere auseinandergerollten, Zustand überführt, vorzugsweise vorgespannt.

In einer weiteren Ausführungsform weist das Kit ferner einen medizinisch verträglichen Kleber auf.

Bezüglich weiterer Merkmale und Vorteile des medizinischen Kits, insbesondere des medizinischen Produktes, wird zur Vermeidung von Wiederholungen vollständig auf die im Rahmen des ersten und zweiten Erfindungsaspektes gemachten Ausführungen Bezug genommen.

Weitere Merkmale und Vorteile der Erfindung ergeben sich aus der nachfolgenden Beschreibung bevorzugter Ausführungsformen in Form von Figuren, Figurenbeschreibungen sowie aus den Unteransprüchen. Die bevorzugten Ausführungsformen dienen dabei lediglich dem besseren Verständnis der Erfindung, ohne diese hierauf zu beschränken.

### Kurze Beschreibung der Figuren

Die Erfindung wird nachfolgend unter Bezugnahme auf die Figuren detailliert beschrieben. Hierbei zeigen schematisch:
- Fig. 1a-d:: ein erfindungsgemäßes medizinisches Produkt sowie dessen Anwendung beim Verschluss von Körpergewebe und
- Fig. 2a-b:: einen Applikator für ein medizinisches Produkt gemäß der vorliegenden Erfindung.

### Detaillierte Beschreibung der Ausführungsbeispiele

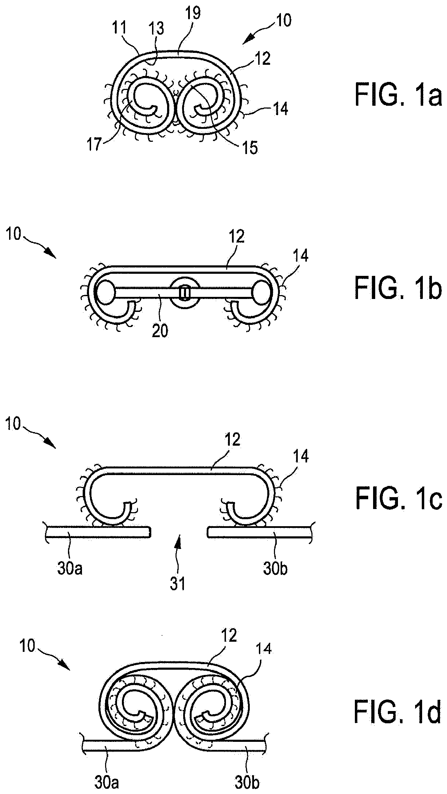

Fig. 1a zeigt schematisch ein medizinisches Produkt 10 gemäß der vorliegenden Erfindung.

Das Produkt 10 weist ein Flächengebilde 12 sowie Verankerungselemente 14 auf.

Das Flächengebilde 12 besitzt zwei gegenüberliegende Seitenflächen 11 und 13. Das Flächengebilde 12 besitzt weiterhin zwei zusammengerollte Teilbereiche 15 und 17, welche über einen nicht zusammengerollten Zwischenbereich 19 voneinander beabstandet sein können.

Das Flächengebilde 12 ist als Netz oder Gitter ausgebildet.

Die Verankerungselemente 14 sind derart ausgebildet, dass sie eine Verankerung des Flächengebildes 12 und damit des Produktes 10 in einem biologischen, insbesondere menschlichen oder tierischen, Gewebe ermöglichen. Hierfür können die Verankerungselemente 14 beispielsweise hakenförmig ausgebildet sein.

Die Verankerungselemente 14 sind lediglich auf der Seitenfläche 11 und dort vorzugsweise nur im Bereich der zusammengerollten Teilbereiche 15 und 17 ausgebildet.

Das Flächengebilde 12 sowie die Verankerungselemente 14 können beispielsweise aus einem nichtrostenden austenitischen Chrom-Nickel-Stahl mit einem Kohlenstoffgehalt von 0.05 Gew.-% bis 0.15 Gew.-%, einem Chromgehalt von 16 Gew.-% bis 19 Gew.-%, einem Nickelgehalt von 6 Gew.-% bis 9.50 Gew.-%, einem Molybdängehalt von maximal 0.80 Gew.-% sowie einem Stickstoffgehalt von maximal 0.11 Gew.-% gebildet sein. Ein solcher Chrom-Nickel-Stahl ist unter der Werkstoffnummer WNr. 1.4310 (X10CrNi18-8) erhältlich.

Fig. 1b zeigt schematisch das erfindungsgemäße Produkt 10 auf einem Spreizapplikator 20. Das Flächengebilde 12 liegt hierbei in einem aufgeweiteten bzw. aufgespreizten, insbesondere auseinandergerollten, und vorzugsweise vorgespannten Zustand vor. In diesem Zustand des Flächengebildes 12 kann eine Applikation des medizinischen Produktes 10 auf zu verschließende Körpergewebebereiche 30a; 30b, die durch einen Gewebespalt 31 voneinander beabstandet sind, erfolgen.

Bei der Applikation des Produktes 10 greifen die Verankerungselemente 14 in die Körpergewebebereiche 30a; 30b ein, wobei sich das Flächengebilde 12 unmittelbar nach Wegnahme des Applikators 20 in einem noch aufgeweiteten bzw. aufgespreizten, insbesondere auseinandergerollten, Zustand befindet (siehe Fig. 1c). Die in diesem Zustand auf das Flächengebilde 12 wirkende Zusammenrollkraft bewirkt ein Zusammenrollen des Flächengebildes 12, wobei die in den Körpergewebebereichen 30a; 30b verankerten Verankerungselemente 14 mitbewegt werden. Dadurch werden die Körpergewebebereiche 30a; 30b ausgehend von ihren freien, d.h. dem Gewebespalt 31 zugewandten, Enden in das Flächengebilde 12 eingerollt und gleichzeitig aneinander fixiert. Dadurch ist ein Verschluss des Gewebespaltes 31 erzielbar (siehe Fig. 1d).

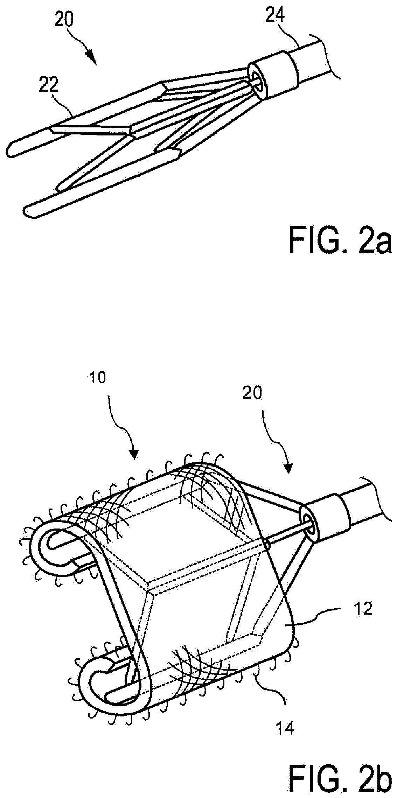

Fig. 2a und 2b zeigen schematisch einen zum Applizieren des erfindungsgemäßen Produktes 10 geeigneten Applikator 20. Dieser weist eine Anlegefläche 22 zum Aufweiten bzw. Auseinanderspreizen, insbesondere Auseinanderrollen, des Flächengebildes 12 sowie einen Schaft 24 auf.

Die Anlegefläche 22 ist auf- und zusammenklappbar ausgestaltet. Zum Aufweiten bzw. Auseinanderspreizen, insbesondere Auseinanderrollen, wird das Flächengebilde 12 auf die in Fig. 2a dargestellte zusammengeklappte Anlegefläche 22 aufgelegt. Eine Aufweitung und vorzugsweise Vorspannung des Flächengebildes 12 erfolgt durch ein nachfolgendes Aufklappen der Anlegefläche 22 (siehe Fig. 2b). In diesem Zustand kann das Produkt 10 auf ein zu verschließendes Körpergewebe appliziert werden. Nach Applikation des Produktes 10 wird die Anlegefläche 22 wieder zusammengeklappt und aus dem Behandlungssitus entfernt. Das Auf- und Zusammenklappen der Anlegefläche 22 kann insbesondere mechanisch oder elektronisch gesteuert werden.

## Patentansprüche

1. Medizinisches Produkt (10), aufweisend ein wenigstens teilweise zusammengerolltes, flexibles Flächengebilde (12), welches unter wenigstens teilweiser Aufhebung einer Zusammenrollkraft in einen aufgeweiteten Zustand überführt werden kann, wobei das Flächengebilde (12) Verankerungselemente (14) zur Verankerung in einem biologischen Gewebe aufweist, wobei es sich bei dem medizinischen Produkt (10) um ein chirurgisches Implantat handelt, wobei das Flächengebilde (12) zwei zusammengerollte Teilbereiche (15;17) aufweist,
**dadurch gekennzeichnet, dass** es sich bei dem Flächengebilde (12) um ein textiles Flächengebilde oder um ein Gitter handelt, und die Verankerungselemente (14) nur auf einer Seitenfläche (11) des Flächengebildes (12) im Bereich der zusammengerollten Teilbereiche (15; 17) ausgebildet sind, wobei die Verankerungselemente (14) auf randseitig verlaufenden Teilflächen der Seitenfläche (11) ausgebildet sind, welche im aufgeweiteten Zustand des Flächengebildes (12) gegenüberliegend angeordnet sind.

2. Medizinisches Produkt (10) nach Anspruch 1, **dadurch gekennzeichnet, dass** die beiden Teilbereiche zylindrisch ausgebildet sind.

3. Medizinisches Produkt (10) nach Anspruch 1 oder 2, **dadurch gekennzeichnet, dass** das Flächengebilde (12) zwischen den beiden Teilbereichen (15;17) einen nicht zusammengerollten Zwischenbereich (19) aufweist.

4. Medizinisches Produkt (10) nach einem der vorhergehenden Ansprüche, **dadurch gekennzeichnet, dass** das Flächengebilde (12) im aufgeweiteten Zustand eine polygonale, vorzugsweise rechteckige, Form besitzt.

5. Medizinisches Produkt (10) nach einem der Ansprüche 1 bis 3, **dadurch gekennzeichnet, dass** das Flächengebilde (12) im aufgeweiteten Zustand eine ovale, insbesondere kreisförmige oder elliptische, Form besitzt.

6. Medizinisches Produkt (10) nach einem der vorhergehenden Ansprüche, **dadurch gekennzeichnet, dass** es sich bei dem textilen Flächengebilde um ein Netz handelt.

7. Medizinisches Produkt (10) nach einem der vorhergehenden Ansprüche, **dadurch gekennzeichnet, dass** das Flächengebilde (12) und/oder die Verankerungselemente (14) ein nicht resorbierbares Polymer aufweisen oder aus einem nicht resorbierbaren Polymer gebildet sind, welches ausgewählt ist aus der Gruppe bestehend aus Polyethylen, Polyethylen mit niedriger Dichte, Polyethylen mit hoher Dichte, hochmolekulares Polyethylen, ultrahochmolekulares Polyethylen, Polypropylen, Polyethylenterephthalat, Polypropylenterephthalat, Polybutylenterephthalat, Polyacrylnitril, Polyamid 6, Polyamid 6-6, Polyamid 6-12, Polyamid 12, Polytetrafluorethylen, insbesondere expandiertes Polytetrafluorethylen, Polyvinylidendifluorid, Polytetrafluorpropylen, Polyhexafluorpropylen, Copolymere davon und Kombinationen, insbesondere Komposite bzw. Verbundmaterialien, davon.

8. Medizinisches Produkt (10) nach einem der Ansprüche 1 bis 6, **dadurch gekennzeichnet, dass** das Flächengebilde (12) und/oder die Verankerungselemente (14) ein resorbierbares Polymer aufweisen oder aus einem resorbierbaren Polymer gebildet sind, welches ausgewählt ist aus der Gruppe bestehend aus Polyglykolsäure, Polymilchsäure, Poly-3-hydroxybuttersäure, Poly-4-hydroxybuttersäure, Poly-ε-caprolacton, Polydioxanon, Polytrimethylencarbonat, Poly-ε-caprolacton, Polyvinylalkohol, Salze davon, Copolymere davon und Kombinationen, insbesondere Komposite bzw. Verbundmaterialien, davon.

9. Medizinisches Produkt (10) nach einem der Ansprüche 1 bis 6, **dadurch gekennzeichnet, dass** das Flächengebilde (12) und/oder die Verankerungselemente (14) ein Metall oder eine Metalllegierung aufweisen oder aus einem Metall oder einer Metalllegierung gebildet sind, welches/welche ausgewählt ist aus der Gruppe bestehend aus Eisen, Chrom, Nickel, Molybdän, Titan, Kobalt, Mangan, Tantal, Nitinol und Legierungen davon.

10. Medizinisches Produkt (10) nach einem der Ansprüche 1 bis 6 oder 9, **dadurch gekennzeichnet, dass** das Flächengebilde (12) und/oder die Verankerungselemente (14) einen Stahl aufweisen oder aus einem Stahl gebildet sind, wobei es sich bei dem Stahl vorzugsweise um einen Chrom-Nickel-Stahl, insbesondere um einen Chrom-Nickelstahl mit einem Chromgehalt von 16 Gew.-% bis 19 Gew.-%, einem Nickelgehalt von 6 Gew.-% bis 9.5 Gew.-% und einem Molybdängehalt von maximal 0.8 Gew.-%, handelt.

11. Medizinisches Produkt (10) nach einem der vorhergehenden Ansprüche, **dadurch gekennzeichnet, dass** die Verankerungselemente (14) in Form von Haken, insbesondere Widerhaken, Ankern, Krallen, Klammern und Kombinationen davon, ausgebildet sind.

12. Medizinisches Produkt (10) nach einem der vorhergehenden Ansprüche, **dadurch gekennzeichnet, dass** die Seitenfläche (11) zwischen den Teilflächen frei von Verankerungselementen ist.

13. Medizinisches Kit, aufweisend ein medizinisches Produkt (10) nach einem der vorhergehenden Ansprüche und wenigstens eine weitere Kitkomponente, welche ausgewählt ist aus der Gruppe bestehend aus einem Applikator (20) für das medizinische Produkt und einem medizinisch verträglichen Kleber.

## Claims

1. Medical product (10), comprising an at least partially rolled, flexible two-dimensional structure (12) which can be transferred to an expanded condition by at least partially overcoming a rolling-up force, wherein the two-dimensional structure (12) includes anchoring elements (14) for anchoring in a biological tissue, wherein the medical product (10) is a surgical implant, wherein the two-dimensional structure (12) includes two rolled-up partial sections (15; 17),
**characterized in that**
the two-dimensional structure (12) is a textile fabric or a mesh, and the anchoring elements (14) are provided only on one side surface (11) of the two-dimensional structure (12) in the region of the rolled-up partial sections (15; 17), wherein the anchoring elements (14) are provided on peripherally extending partial surfaces of the side surface (11), which partial surfaces are in opposite arrangement when the two-dimensional structure (12) is in the expanded condition.

2. Medical product (10) according to claim 1, **characterized in that** the two partial sections are cylindrical.

3. Medical product (10) according to claim 1 or 2, **characterized in that** the two-dimensional structure (12) has a non-rolled-up intermediate section (19) between the two partial sections (15; 17).

4. Medical product (10) according to any of the preceding claims, **characterized in that** the two-dimensional structure (12) in the expanded condition has a polygonal, preferably rectangular, shape.

5. Medical product (10) according to any of claims 1 to 3, **characterized in that** the two-dimensional structure (12) in the expanded condition has an oval, in particular circular or elliptic, shape.

6. Medical product (10) according to any of the preceding claims, **characterized in that** the textile fabric is a netted mesh.

7. Medical product (10) according to any of the preceding claims, **characterized in that** the two-dimensional structure (12) and/or the anchoring elements (14) comprise/comprises a non-absorbable polymer or are/is made of a non-absorbable polymer which is selected from the group consisting of polyethylene, low-density polyethylene, high-density polyethylene, high-molecular-weight polyethylene, ultrahigh-molecular-weight polyethylene, polypropylene, polyethylene terephthalate, polypropylene terephthalate, polybutylene terephthalate, polyacrylonitrile, polyamide 6, polyamide 6-6, polyamide 6-12, polyamide 12, polytetrafluoroethylene, in particular expanded polytetrafluoroethylene, polyvinylidene difluoride, polytetrafluoropropylene, polyhexafluoropropylene, copolymers thereof and combinations thereof, in particular composites or composite materials thereof.

8. Medical product (10) according to any of claims 1 to 6, **characterized in that** the two-dimensional structure (12) and/or the anchoring elements (14) comprise/comprises a resorbable polymer or are/is made of a resorbable polymer which is selected from the group consisting of polyglycolic acid, polylactic acid, poly-3-hydroxybutyric acid, poly-4-hydroxybutyric acid, poly-ε-caprolactone, polydioxanone, polytrimethylene carbonate, poly-ε-caprolactone, polyvinyl alcohol, salts thereof, copolymers thereof and combinations thereof, in particular composites or composite materials thereof.

9. Medical product (10) according to any of claims 1 to 6, **characterized in that** the two-dimensional structure (12) and/or the anchoring elements (14) comprise/comprises a metal or a metallic alloy or are/is made of a metal or a metallic alloy which is selected from the group consisting of iron, chromium, nickel, molybdenum, titanium, cobalt, manganese, tantalum, nitinol and alloys thereof.

10. Medical product (10) according to any of claims 1 to 6 or 9, **characterized in that** the two-dimensional structure (12) and/or the anchoring elements (14) comprise/comprises a steel or are/is made of a steel, wherein the steel is preferably a chromium-nickel steel, in particular a chromium-nickel steel including a chromium content of 16% by weight to 19% by weight, a nickel content of 6% by weight to 9.5% by weight, and a molybdenum content of at maximum 0.8 % by weight.

11. Medical product (10) according to any of the preceding claims, **characterized in that** the anchoring elements (14) are provided in the form of hooks, in particular barbs, anchors, claws, clamps and combinations thereof.

12. Medical product (10) according to any of the preceding claims, **characterized in that** the side surface (11) between the partial surfaces is free of anchoring elements.

13. Medical kit, comprising a medical product (10) according to any of the preceding claims and at least one further kit component which is selected from the group consisting of an applicator (20) for the medical product and a medically compatible adhesive.

## Revendications

1. Produit médical (10), présentant un article plat flexible (12), au moins partiellement enroulé, qui peut être amené dans un état déployé en supprimant au moins partiellement une force d'enroulement, dans lequel l'article plat (12) présente des éléments d'ancrage (14) destinés à l'ancrage dans un tissu biologique, dans lequel le produit médical (10) est un implant chirurgical, dans lequel l'article plat (12) présente deux zones partielles enroulées (15; 17), **caractérisé en ce que** l'article plat (12) est un article plat textile ou un treillis, et les éléments d'ancrage (14) ne sont formés que sur une face latérale (11) de l'article plat (12) dans la région des zones partielles enroulées (15; 17), dans lequel les éléments d'ancrage (14) sont formés sur des surfaces partielles formées en bordure des faces latérales (11), qui sont disposées l'une en face de l'autre dans l'état déployé de l'article plat (12).

2. Produit médical (10) selon la revendication 1, **caractérisé en ce que** les deux zones partielles sont de forme cylindrique.

3. Produit médical (10) selon la revendication 1 ou 2, **caractérisé en ce que** l'article plat (12) présente entre les deux zones partielles (15; 17) une zone intermédiaire non enroulée (19).

4. Produit médical (10) selon l'une quelconque des revendications précédentes, **caractérisé en ce que** l'article plat (12) présente dans l'état déployé une forme polygonale, de préférence rectangulaire.

5. Produit médical (10) selon l'une quelconque des revendications 1 à 3, **caractérisé en ce que** l'article plat (12) présente dans l'état déployé une forme ovale, en particulier circulaire ou elliptique.

6. Produit médical (10) selon l'une quelconque des revendications précédentes, **caractérisé en ce que** l'article plat textile est un filet.

7. Produit médical (10) selon l'une quelconque des revendications précédentes, **caractérisé en ce que** l'article plat (12) et/ou les éléments d'ancrage (14) présentent un polymère non résorbable ou sont formés en un polymère non résorbable, qui est choisi dans le groupe comprenant le polyéthylène, le polyéthylène à faible densité, le polyéthylène à haute densité, le polyéthylène à haut poids moléculaire, le polyéthylène à ultra-haut poids moléculaire, le polypropylène, le téréphtalate de polyéthylène, le téréphtalate de polypropylène, le téréphtalate de polybutylène, le polyacrylonitrile, le polyamide 6, le polyamide 6-6, le polyamide 6-12, le polyamide 12, le polytétrafluoroéthylène, en particulier le polytétrafluoroéthylène expansé, le difluorure de polyvinylidène, le polytétra-fluoropropylène, le polyhexafluoropropylène, des copolymères de ceux-ci et des combinaisons, en particulier des composites ou des matériaux composites, de ceux-ci.

8. Produit médical (10) selon l'une quelconque des revendications 1 à 6, **caractérisé en ce que** l'article plat (12) et/ou les éléments d'ancrage (14) présentent un polymère résorbable ou sont formés en un polymère résorbable, qui est choisi dans le groupe comprenant l'acide polyglycolique, l'acide polylactique, l'acide poly-3-hydroxybutyrique, l'acide poly-4-hydroxy-butyrique, la poly-ε-caprolactone, le polydioxanone, le carbonate de polytriméthylène, la poly-ε-caprolactone, l'alcool polyvinylique, des sels de ceux-ci, des copolymères de ceux-ci, des combinaisons, en particulier des composites ou des matériaux composites, de ceux-ci.

9. Produit médical (10) selon l'une quelconque des revendications 1 à 6, **caractérisé en ce que** l'article plat (12) et/ou les éléments d'ancrage (14) présentent un métal ou un alliage métallique ou sont formés en un métal ou en un alliage métallique, qui est choisi dans le groupe comprenant le fer, le chrome, le nickel, le molybdène, le titane, le cobalt, le manganèse, le tantale, le Nitinol et des alliages de ceux-ci.

10. Produit médical (10) selon l'une quelconque des revendications 1 à 6 ou 9, **caractérisé en ce que** l'article plat (12) et/ou les éléments d'ancrage (14) présentent un acier ou sont formés en un acier, dans lequel l'acier est de préférence un acier au chrome-nickel, en particulier un acier au chrome-nickel ayant une teneur en chrome de 16 % en poids à 19 % en poids, un teneur en nickel de 6 % en poids à 9,5 % en poids et une teneur en molybdène de 0,8 % en poids au maximum.

11. Produit médical (10) selon l'une quelconque des revendications précédentes, **caractérisé en ce que** les éléments d'ancrage (14) sont réalisés sous forme de crochets, en particulier de barbes, d'ancres, de griffes, d'agrafes et de combinaisons de ceux-ci.

12. Produit médical (10) selon l'une quelconque des revendications précédentes, **caractérisé en ce que** la face latérale (11) entre les surfaces partielles est dépourvue d'éléments d'ancrage.

13. Kit médical, présentant un produit médical (10) selon l'une quelconque des revendications précédentes et au moins un autre composant de kit, qui est choisi dans le groupe composé d'un applicateur (20) pour le produit médical et d'une colle médicalement compatible.
